# EUROPEAN PATENT APPLICATION

(11) **EP 1 985 299 A1**
(43) Date of publication of application: **29.10.2008**
(21) Application number: 07706848.4
(22) Date of filing: 16.01.2007
(51) Int. Cl.: A61K 31/702, A61K 31/737, A61P 25/00, C07H 11/00, C08B 37/00

(54) **THERAPEUTIC AGENT FOR TRAUMATIC NEUROPATHY AND/OR MOVEMENT DISORDER**

(30) Priority: 17.01.2006 JP 2006009215
(71) Applicant: Glycoscience Laboratories, Inc., Tokyo 113-8549 (JP)
(72) Inventor: ASARI, Akira, Tokyo 113-8549 (JP); KATO, Tadahiko, Musashimurayama-shi, Tokyo 208-0011 (JP)
(74) Representative: Wolff, Felix
(86) International application number: PCT/JP2007/050521
(87) International publication number: WO 2007/083634

(57) **Abstract**

The object is to provide a therapeutic drug for traumatic neural disease (disorder) and/or motor function disorder, more particularly, a therapeutic drug for traumatic neural disease (disorder) and/or motor function disorder derived from spinal cord injury. A keratan sulfate oligosaccharide or a derivative thereof was found to have an effect of improving the traumatic neural disease (disorder) and/or motor function disorder derived from spinal cord injury and to be useful as the therapeutic drug for traumatic neural disease (disorder) and/or motor function disorder. That i.s, according to the present invention, there is provided a therapeutic drug for traumatic neural disease (disorder) and/or motor function disorder comprising an effective amount of the keratan sulfate oligosaccharide or the derivative thereof.

## Description

### FIELD OF THE INVENTION

The present invention relates to a therapeutic drug for traumatic neural disease (disorder) and/or motor function disorder, and particularly to a therapeutic drug for traumatic neural disease (disorder) and/or motor function disorder derived from spinal cord injury.

### BACKGROUND OF THE INVENTION

Asari et al. reported that L4, a keratan sulfate disaccharide, represented by the following formula has an inhibitory effect on interleukin-12 (IL-12) expression (Xu H et al. (2005)):
Gal(6S)βl-4GlcNAc(6S)
wherein Gal represents a galactose residue; GlcNAc represents an N-acetylglucosamine residue; 6S means that the hydroxyl group at position 6 is in the form of 6-0-sulfate ester; and β1-4 represents a β1-4-glycosidic bond.

It is known that IL-12 is expressed at a high level in MRL-lpr/lpr mouse, immortalizes T lymphocytes (CD4-CD8-), and causes lymphatic organs (lymph node and spleen) to swell (Xu H et al. (2001)). Additionally, it has been reported that administration of L4 reduces IL-12 concentration in the blood, induces cell death of T lymphocytes, and causes shrinking of lymphatic organs (Xu H et al. (2005)).

On the other hand, it has been reported that semen of patients with spinal cord injury contains high-level IL-12 (Basu S et al). However, the role of IL-12 in spinal cord injury is yet unknown.

### REFERENCES

[Patent document 1] Japanese Patent Application Unexamined Publication No. H2-57182 (1990)
[Patent document 2] International Publication WO 96/16166 pamphlet
[Patent document 3] International Publication WO 96/16973 pamphlet
[Patent document 4] Japanese Patent Application Unexamined Publication No. 2001-89493
[Non-patent document 1] Xu H, Kurihara H, Ito T, Kikuchi H, Yoshida K, Yamanokuchi H, Asari A. The keratan sulfate disaccharide Gal(6SO3) beta-1,4-GlcNAc(6SO3) modulates interleukin 12 production by macrophages in murine Thy-1 type autoimmune disease. J. Biol. Chem. 2005 May 27;280(21):20879-86.
[Non-patent document 2] Xu H, Kurihara H, Ito T, Nakajima S, Hagiwara E, Yamanokuchi H, Asari A. IL-12 enhances lymphoaccumulation by suppressing cell death of T cells in MRL-lpr/lpr mice. J. Autoimmun. 2001 Mar;16(2):87-95.
[Non-patent document 3] Basu S, Aballa TC, Ferrell SM, Lynne CM, Brackett NL. Inflammatory cytokine concentrations are elevated in seminal plasma of men with spinal cord injuries. J. Androl. 2004 Mar-Apr;25(2):250-4.
[Non-patent document 4] Kubota, M., Yoshida, K., Tawada, A., and Ohashi, M. (2000) Eur. J. Mass Spectrom. 6, 193-203.
[Non-patent document 5] Basso DM, Beattie MS, Bresnahan JC. A sensitive and reliable locomotor rating scale for open field testing in rats. J. Neurotauma, 1995 Feb, 12(1), pl-21.
[Non-patent document 6] Basso DM, Beattie MS, Bresnahan JC. Graded histological and locomotor outcomes after spinal cord contusion using the NYU weight-drop device versus transection. Exp. Neurol., 1996 Jun, 139(2), p244-56.

### SUMMARY OF THE INVENTION

### Technical Problem

The object of the present invention is to provide a therapeutic drug for traumatic neural disease (disorder) and/or motor function disorder, and particularly a therapeutic drug for traumatic neural disease (disorder) and/or motor function disorder derived from spinal cord injury.

### Solution of Problem

According to one aspect of the present invention, there is provided a therapeutic drug for traumatic neural disease (disorder) comprising an effective amount of a keratan sulfate oligosaccharide or a derivative thereof. According to another aspect of the present invention, there is provided a therapeutic drug for motor function disorder comprising an effective amount of a keratan sulfate oligosaccharide or a derivative thereof.

### Advantageous Effects of Invention

The present inventors have studied the effect of keratan sulfate oligosaccharides or derivatives thereof on spinal cord injury using a rat spinal cord injury model. As a result, it was found as described below in detail that, surprisingly, keratan sulfate oligosaccharides or derivatives thereof provide an improvement effect on motor function disorder caused by spinal cord injury and are useful as a therapeutic drug for traumatic neural disease (disorder) and/or motor function disorder.

### BRIEF DESCRIPTION OF THE DRAWINGS

FIG. 1 is a graph showing an effect of L4 on hind limb motor function in a rat spinal cord injury model according to a crush method with forceps, where a relation between the hind limb motor function (BBB score) and days after treatment of spinal cord injury and administration of L4 is shown;
FIG. 2 is a graph showing the effect of L4 on the hind limb motor function in the rat spinal cord injury model according to the crush method with forceps, where the hind limb motor function (BBB score) on the seventh day after the treatment of spinal cord injury and the administration of L4 is shown; and
FIG. 3 is a graph showing an effect of L4 on spinal cord evoked potential in the rat spinal cord injury model according to the crush method with forceps, where the spinal cord evoked potential on the seventh day after the treatment of spinal cord injury and the administration of L4 is shown.

### DEATILED DESCRIPTION OF PREFERRED EMBODIMENTS

Hereinafter, the embodiments of the present invention are explained. However, the present invention is not limited by the embodiments explained below.

As described above, according to the present invention, there is provided a therapeutic drug for traumatic neural disease (disorder) and/or motor function disorder comprising an effective amount of a keratan sulfate oligosaccharide or a derivative thereof.

In the present invention, "keratan sulfate oligosaccharide" (hereinafter, also referred to as KS oligosaccharide) is an oligosaccharide of di- or higher saccharide comprising the basic structure of keratan sulfate (generally refers to, but not limited to, a structure in which a galactose residue or galactose-6-O-sulfate residue and an N-acetylglucosamine residue or N-acetylglucosamine-6-O-sulfate residue are alternately linked by glycosidic bonds). Further, KS oligosaccharide may contain a sialic acid (acyl derivative of neuraminic acid) residue(s) and/or a fucose residue(s). Usually, sialic acid residues are linked to galactose residues via α-2,3-or α-2,6-glycosidic bond, and fucose residues are linked to N-acetylglucosamine residues via α-1,3-glycosidic bond.

The oligosaccharide in the present invention generally comprises constituent sugar substances referred to as oligosaccharides in the field of polysaccharide. Particularly, the oligosaccharide is preferably a sugar polymer of from two to 10 sugar units, more preferably from two to six sugar units, and further more preferably from two to four sugar units. Among these, an oligosaccharide of two sugar units is particularly preferred.

The KS oligosaccharide may be a product obtainable, for example, by enzymatic or chemical degradation of a keratan sulfate and may also be a compound obtainable, for example, by sulfation of an oligosaccharide in which one or more N-acetyllactosamine units are linked. Among such keratan sulfate oligosaccharides, an oligosaccharide obtainable by degradation of keratan sulfate (oligosaccharide derived from keratan sulfate) is preferable, and a degradation product obtainable by degradation of keratan sulfate with an endo-β-N-acetylglucosaminidase type keratan sulfate hydrolase is more preferable.

In keratan sulfate of natural origin, many of the hydroxyl groups at position 6 of N-acetylglucosamine residues are sulfated, and part of the hydroxyl groups at position 6 of galactose residues are usually sulfated. Although a KS oligosaccharide may be a keratan sulfate oligosaccharide obtained by degradation of this keratan sulfate as described above, the KS oligosaccharide of the present invention is one in which the hydroxyl groups at position 6 of N-acetylglucosamine residues may be either sulfated or not and preferably, the hydroxyl groups at position 6 of galactose residues are sulfated.

In other words, the KS oligosaccharide is preferably either one of the disaccharides represented by the following formulae or an oligosaccharide of even-numbered sugars containing either or both of the disaccharides as repeating structural units:
Gal(6S)-GlcNAc(6S) and
Gal(6S)-GlcNAc
wherein Gal represents a galactose residue; GlcNAc represents an N-acetylglucosamine residue; 6S means that the hydroxyl group at position 6 is in the form of 6-O-sulfate ester; and - represents a glycosidic bond.

The KS oligosaccharide is more preferably any one of the oligosaccharides represented by the following formulae 1 to 3:

Gal(6S)β1-4GlcNAc(6S)β1-3Gal(6S)βl-4GlcNAc(6S) Formula 1

Gal(6S)β1-4GlcNAc(6S) Formula 2

Gal(6S)β1-4GlcNAc Formula 3

wherein Gal represents a galactose residue; GlcNAc represents an N-acetylglucosamine residue; 6S means that the hydroxyl group at position 6 is in the form of 6-O-sulfate ester; β1-4 represents a β1-4 glycosidic bond; and β1-3 represents a βl-3 glycosidic bond.

Hereinafter, the oligosaccharide represented by the formula 1 is also referred to as L4L4, the oligosaccharide represented by the formula 2 as L4, and the oligosaccharide represented by the formula 3 as L3. The oligosaccharides represented by the formulae 1 to 3 described above may be linked with a sialic acid residue such as N-acetylneuraminic acid, and the following examples are listed as keratan sulfate oligosaccharides containing such a sialic acid:
SA-Gal(6S)-GlcNAc(6S) and
SA-Gal(6S)-GlcNAc(6S)-Gal(6S)-GlcNAc(6S)
wherein SA represents a sialic acid residue; Gal represents a galactose residue; GlcNAc represents an N-acetylglucosamine residue; 6S means that the hydroxyl group at position 6 is in the form of 6-O-sulfate ester; and - represents a glycosidic bond.

In the present specification, a "derivative" of the keratan sulfate oligosaccharide (hereinafter, also referred to as KS oligosaccharide derivative) usually comprises one in which at least one of hydrogen atoms of hydroxyl groups in a KS oligosaccharide (preferably 10 percent or more of all hydroxyl groups) is replaced by an acyl group (partially or totally O-acylated derivative).

Additionally, the KS oligosaccharide and the KS oligosaccharide derivative comprise ones in an ionized state as well as ones having a structure in which a proton or cation is added. Therefore, the (cation adduct) KS oligosaccharide and KS oligosaccharide derivative include pharmaceutically acceptable salts thereof.

The pharmaceutically acceptable salts comprise, but are not limited to, those that are pharmaceutically acceptable among alkaline metal salts such as sodium salt, potassium salt, and lithium salt; alkaline earth metal salts such as calcium salt; salts formed with inorganic bases such as ammonium salt; and salts formed with organic bases such as diethanolamine salt, cyclohexylamine salt, and amino acid salt.

In the KS oligosaccharide derivative, the acyl group substituting for a hydrogen atom of hydroxyl group of the KS oligosaccharide is preferably an acyl group of 1 to 10 carbon atoms, more preferably an aliphatic or aromatic acyl group of 1 to 10 carbon atoms, that is, alkanoyl group or aroyl group that may contain a hetero atom. Examples of these groups include acetyl, chloroacetyl, dichloroacetyl, trifluoroacetyl, methoxyacetyl, propionyl, n-butyryl, (E)-2-methylbutenoyl, isobutyryl, pentanoyl, benzoyl, o-(dibromomethyl)benzoyl, o-(methoxycarbonyl)benzoyl, 2,4,6-trimethylbenzoyl, p-toluoyl, p-anisoyl, p-chlorobenzoyl, p-nitrobenzoyl, and the like. When the KS oligosaccharide derivative has a plurality of acyl groups, these acyl groups may be mutually the same or different. When the hydrogen atom of the hydroxyl group at position 1 of the reducing end sugar of the KS oligosaccharide is substituted by an acyl group, the configuration of O-acyl group may be either in α-glycosidic configuration or β-glycosidic configuration, but is preferably α-glycosidic configuration.

The acylated KS oligosaccharide is particularly preferable because of advantages such as improved solubility in organic solvents and lipids, enhanced biomembrane permeability, and increased gastrointestinal absorption when administered orally.

In the present invention, the KS oligosaccharide or the KS oligosaccharide derivative is preferably a compound represented by a formula 4 below. For convenience, the formula 4 shows a structure in which M is added. However, the compound may be ionized in a solution, and when ionized, the sulfonic acid group turns into the state of the negative ion. 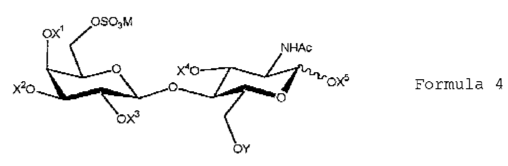 wherein X¹ to X⁵ each independently represent a hydrogen atom or an acyl group; Y represents a hydrogen atom or SO₃M; M represents a hydrogen atom, a mono- to trivalent metal cation, or a mono- to trivalent base; and the bond shown by a wavy line represents a single bond in α-glycosidic configuration or β-glycosidic configuration.

The KS oligosaccharide substituted by acyl group is preferably one in which all of X¹ to X⁵ in the above formula 4 are acetyl groups. Particularly preferred is a derivative shown by a formula 5 below. 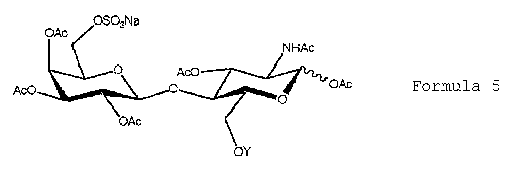 wherein Ac represents an acetyl group; Y represents a hydrogen atom or SO₃Na; and the bond shown by a wavy line represents a single bond in α-glycosidic configuration or β-glycosidic configuration.

The therapeutic drug according to the present invention may comprise a single species or a mixture of the KS oligosaccharide or derivative thereof, for example, comprise either a substance in α-glycosidic configuration or a substance in β-glycosidic configuration, or a mixture of these substances with respect to the part shown by the wavy line in the above formula 4.

For example, the KS oligosaccharide can be obtained by allowing a buffered solution of a keratan sulfate, preferably a highly sulfated keratan sulfate, to be digested by treatment with an endo-β-N-acetylglucosaminidase type keratan sulfate hydrolase, e.g. keratanase II derived from a bacterium belonging to Bacillus (JP H2-57182 A (1990)) or a keratan sulfate hydrolase derived from Bacillus circulans KsT202 strain (WO 96/16166), followed by fractionation of the resulted digested products. By subjecting the obtained oligosaccharides to conventional isolation and purification methods, e.g. ethanol precipitation, gel filtration, and anion exchange chromatography, a desired oligosaccharide can be isolated and purified.

Such production methods are disclosed in WO 96/16973. The keratan sulfate serving as a raw material may be composed of repeating units of a disaccharide mainly formed from galactose or galactose-6-O-sulfate and N-acetylglucosamine or N-acetylglucosamine-6-O-sulfate. Although the rate of sulfation in keratan sulfate varies depending on animal species, organs, and the like, keratan sulfate produced from biological raw materials such as cartilaginous fish, e.g. a shark, and cartilage, bone, and cornea of mammals, e.g. whale and bovine can be commonly used.

The keratan sulfate used as the raw material can be any of those readily obtained and is not particularly limited, but is preferably a highly sulfated keratan sulfate in which the constituent sugar, galactose residue, is sulfated (a highly sulfated keratan sulfate containing 1.5 to 2 moles of sulfate group per constituent disaccharide unit is sometimes called keratan polysulfate). Further, the position of the sulfate group in galactose residue is preferably position 6. Such a highly sulfated keratan sulfate can be obtained, for example, from proteoglycan of cartilaginous fish such as a shark, and a commercially available product thereof can also be used.

The KS oligosaccharide thus obtained can be subjected to a known method for desulfating or sulfating sugar chain to adjust the content of sulfate groups for use as the KS oligosaccharide of the present invention.

When producing the KS oligosaccharide derivative, hydrogen atoms of hydroxyl groups in a keratan sulfate can be substituted by acyl groups in accordance with an acylation method commonly performed to protect sugar hydroxyl groups. For example, acyl groups can be introduced in a conventional manner by allowing a KS oligosaccharide to react with a reactive derivative of an acyl group to be introduced (carboxylic acid anhydride (for example, acetic anhydride when introducing acetyl group, and propionic anhydride when introducing propanoyl group), carboxylic acid halide, and the like, each corresponding to the acyl group) in an appropriate solvent (pyridine, dioxane, tetrahydrofuran, N,N-dimethylformamide (DMF), acetonitrile, chloroform, dichloromethane, methanol, ethanol, water, a mixture thereof, etc.). The reaction can also be carried out as necessary in the presence of a base catalyst such as pyridine.

The extent of acylation may also be controlled as needed, and this control can be performed either by partial acylation in the above acylation method or by partial removal of acyl groups from the acylated KS oligosaccharide. The removal of acyl groups can be carried out by hydrolysis with methanolic ammonia, concentrated ammonia water, sodium methoxide, sodium ethoxide, sodium hydroxide, potassium hydroxide, and the like. The obtained derivative can be purified by reverse phase high performance liquid chromatography and the like.

Preferably, the active ingredient of the therapeutic drug according to the present invention, the KS oligosaccharide or its derivative, is purified to an extent sufficient for pharmaceutical use and does not contain contaminants unacceptable therefor.

The therapeutic drug according to the present invention is useful for treatment, inhibition of progression, or prevention of traumatic neural disease (disorder) and/or motor function disorder in mammals such as humans, dogs, bovines, horses, mice, rats, and the like. Particularly, the therapeutic drug according to the present invention is suitable when motor function disorder is derived from neuropathy. Additionally, the therapeutic drug according to the present invention is suitable when neuropathy is derived from spinal cord injury. Spinal cord injury includes traumatic spinal cord injury (luxation or subluxation of vertebral joints, vertebral fracture (linear fracture, compression fracture, crush fracture), complete transverse injury, incomplete transverse injury, Brown-Sequard injury, acute anterior spinal cord injury, acute central spinal cord injury, high cervical spinal cord injury, etc.), vertebral degenerative disease (spondylosis, etc.), inflammatory spinal disease (spondylitis, chronic rheumatoid arthritis, etc.), tumor (spinal cord tumor, vertebral tumor, etc.), vascular disease (spinal apoplexy, spinal paralysis due to disorder of extramedullary vessels, etc.), myelitis (arachnoiditis, viral myelitis, bacterial myelitis, etc.), multiple sclerosis, amyotrophic lateral sclerosis, and the like. The neuropathy derived from spinal cord injury includes motor function disorder of lower-body, hemiplegia, hemiparesis, perception disorder, autonomic nervous system dysfunction, loss of reflection, hypogonadism, and the like. The motor function disorder derived from neuropathy includes shock based on neuropathy, respiratory paralysis, sensory paralysis, motor paralysis, loss of reflection, autonomic nerve paralysis, and the like. It should be noted that the therapeutic drug according to the present invention can be applied not only for curative therapy but also for disease prevention, maintenance (prevention of deterioration), relief (symptom improvement), and the like.

In the present invention, any formulation can be appropriately selected depending on nature and state of progression of a target disease, method of administration, and the like. The therapeutic drug of the present invention can be administered by injection (intravenous, intramuscular, subcutaneous, intracutaneous, intraperitoneal, etc.), nasal, oral, or percutaneous administration, inhalation, or the like, and can be appropriately formulated depending on these administration methods. Applicable formulations can be widely selected, for example, from, but not limited to, injection (solution, suspension, emulsion, solid for preparation just before use, etc.), tablet, capsule, granule, powder, liquid, liposomal formulation, ointment, plaster, lotion, dermatologic paste, patch, gel preparation, suppository, external powder, spray, inhalant, and the like. Additionally, when preparing these formulations, additives that are generally used in drugs, for example, common excipient, stabilizer, binder, lubricant, emulsifier, osmotic regulator, pH controlling agent, and in addition, coloring agent, disintegrating agent, and the like can be used.

Although the content in a formulation as well as the dose of the active ingredient of the therapeutic drug of the present invention, the KS oligosaccharide or derivative thereof, are matters to be determined individually depending on administration method, dosage form, and purpose of use of the drug preparation, patient's specific condition, patient's weight, age, and gender, and the like. The dose in clinical use of the KS oligosaccharide is typically shown to be 50 to 5000 mg once a day for an adult, but is not particularly limited.

The safety of the active ingredient of the therapeutic drug of the present invention, the KS oligosaccharide, is disclosed in WO 96/16973 and the safety of its derivative can be inferred from the examples described in JP 2001-89493 A.

### EXAMPLES

Hereinafter, examples of the present invention are explained with reference to the accompanying drawings. However, the present invention is not limited to the examples explained below.

### [Materials and methods]

### Preparation of L4

As described below, L4 was prepared by degrading keratan sulfate with keratanase II and subsequent fractionation on anion exchange chromatography according to the method of Xu et al. (Xu H et al. (2005)).

KS oligosaccharide (L4) was isolated from the degradation products of keratan sulfate (Seikagaku Corp.) derived from shark fin, produced by keratanase II by using a series of steps of anion exchange chromatography and gel permeation chromatography. The oligosaccharide was identified by capillary electrophoresis and mass spectrometry (Kubota, M).

The capillary electrophoresis was performed using a Quanta 4000 capillary electrophoresis system (Waters) with an ultra violet detector. The capillary electrophoresis system was used in normal polarity with a sample loaded at the anode. As a running buffer, 50 mM sodium tetraborate (pH 9.0) was used. The sample was separated with the use of a quartz glass capillary tube obtained from Waters Corp. (the outside of the tube was covered except for the region where the sample passes through the detector: Inner diameter, 75 µm; length, 60 cm) and analyzed. Prior to sample application, the capillary tube was manually rinsed with 0.5 M sodium hydroxide, distilled water, and the running buffer. The sample was loaded over an injection time of 10 sec under hydrostatic pressure. The experiment was carried out at a constant voltage (12 kV). Eluates were monitored at 185 nm. The analysis of data was performed with a software program, Millenium 32 (version 3.06.01) of Waters Corp.

The oligosaccharide thus obtained was examined for endotoxin by the Limulus amebocyte lysate assay using a Toxicolor LS set (Seikagaku Corp.). Endotoxin contained in L4 was equal to or lower than 0.03 pg/mg.

### Preparation of spinal cord injury model and administration of test substance

A spinal cord injury model (crush model with forceps) was prepared as described below. Twelve-week-old male Wistar rats were used as experimental animals. The animals were shaved from neck to hip with an electric clipper under pentobarbital anesthesia (50 mg/kg body weight) and the skin was cleansed with 70% ethanol and Isodine (product of Meiji Seika Kaisha, Ltd.). After dorsal skin incision, the thoracic spine from T5 to T10 was exposed, the sixth thoracic spine (T6 thoracic spine) was subjected to hemi-laminectomy, and a small incision was made on the dura. Following local anesthesia with Xylocaine (Astra

Zeneca), a forceps (with the tip trimmed to 0.3 mm) was inserted at the position of T6 until its tip reached the vertebral body, and the spinal cord was crushed by sandwiching it from both sides for 10 sec.

Immediately after the injury, the tip of a tube (OD: 0.3 mm) was placed under the dura on the head side of the injured site, and L4 (6 µl) was administered into the dura using a microsyringe (25 µl, Ito Corp.). Gelatin sponge (Gelform, product of Pharmacia Corp.) was placed at the injured site in order to separate from peripheral tissues, the incision wound was sutured, and the rat was returned to a rearing cage.

### Behavioral evaluation

After injury of the spinal cord, the hind limb motor function was assessed daily for 7 days by two independent blind examiners according to the method of Basso et al. (refer to Basso DM et al (1995), (1996)) using Basso-Beeattie-Bresnahan (BBB) Scale, and their average score was taken as a final assessment score. Here, the BBB scale is a measure used in a method for assessing recovery of hind limb motor function by using a finely graded 21-point scale and allowing animals to walk freely in an open field. When spontaneous motor activity of the hind limbs is not observed at all, the score is 0, and when normal motor activity of the hind limbs is observed, the score is 21.

### Measurement of spinal cord evoked potential

On the seventh day after the injury of the spinal cord, endotracheal intubation was performed into the experimental animal under halothane anesthesia (4.0% at the beginning, 1.0% during maintenance). A muscle relaxant was given to the experimental animal to prevent movement and its head was fixed in the prone position, followed by maintenance on a respirator. Catheter electrodes were inserted into the experimental animal from the space between the second/third cervical vertebrae and the space between the 13th thoracic vertebra/first lumbar vertebra, and spinal cord evoked potential (SCEP) was measured with an electromyograph (Counterpoint, Dantec) after applying a supramaximal stimulus (stimulus frequency; 1 Hz, duration; 0.05 msec). The evaluation of the obtained potential was performed with the amplitude of the first potential as an indication. A rise in the spinal cord evoked potential implies a recovery of the injured site.

### Statistical evaluation

The BBB score was evaluated using Tukey's nonparametric multiple comparison test.

### Results

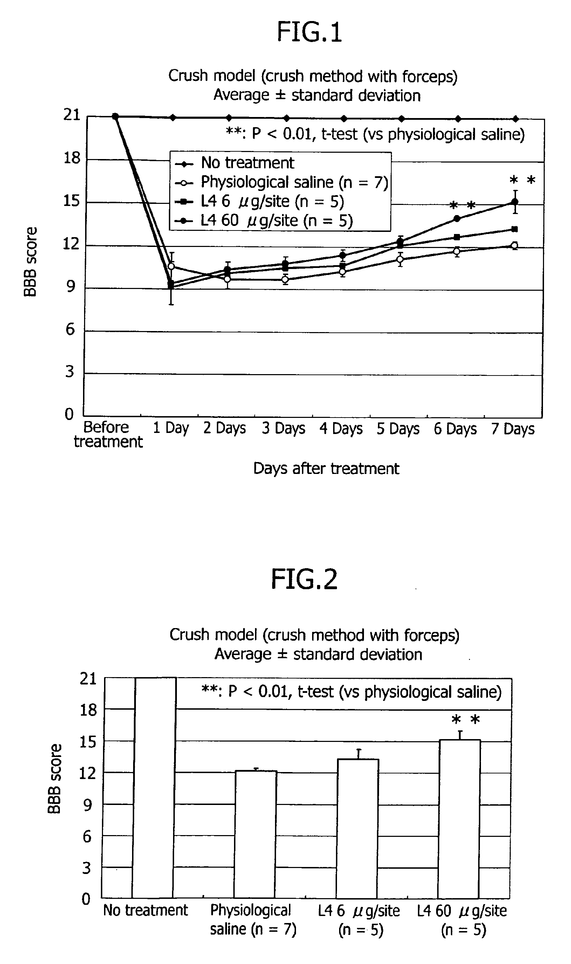

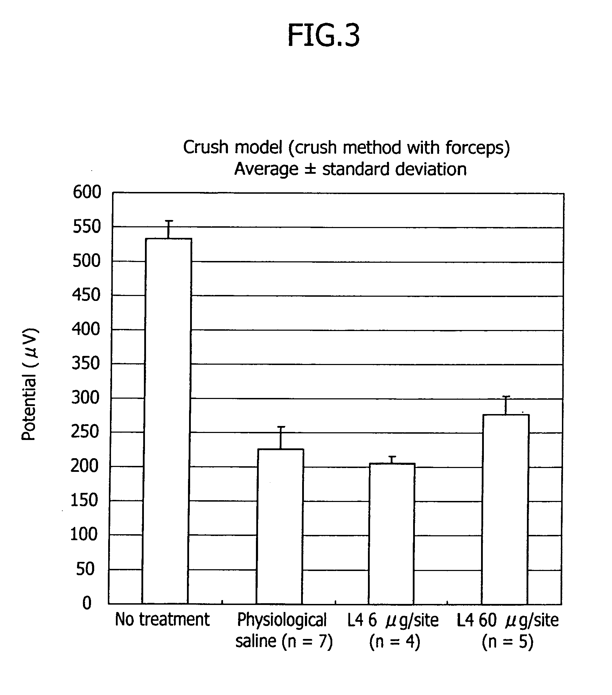

In the test for hind limb motor function using the BBB scale, a significant recovery in hind limb motor function (P < 0.001) was observed on the seventh day after the spinal cord injury by administration of L4 at 60 µg/animal compared with the physiological saline-administered group (FIGS. 1 and 2). In the spinal cord evoked potential, a tendency for a recovery in the SCEP amplitude was observed by administration of L4 at 60 µg/animal compared with the saline-administered group (FIG. 3).

## Claims

1. A therapeutic drug for traumatic neural disease (disorder) comprising an effective amount of a keratan sulfate oligosaccharide or a derivative thereof.

2. A therapeutic drug for motor function disorder comprising an effective amount of a keratan sulfate oligosaccharide or a derivative thereof.

3. The therapeutic drug according to claim 2, wherein the motor function disorder is derived from neuropathy.

4. The therapeutic drug according to claim 1 or 3, wherein the neuropathy is derived from spinal cord injury.

5. The therapeutic drug according to any one of claims 1 to 4, wherein the keratan sulfate oligosaccharide is one of disaccharides represented by the following formulae or an oligosaccharide of even-numbered sugars containing one or both of disaccharides represented by the following formulae as repeating structural units:
Gal(6S)-GlcNAc(6S) and
Gal(6S)-GlcNAc
wherein Gal represents a galactose residue; GlcNAc represents an N-acetylglucosamine residue; 6S means that a hydroxyl group at position 6 is in the form of 6-O-sulfate ester; and - represents a glycosidic bond.

6. The therapeutic drug according to claim 5, wherein the keratan sulfate oligosaccharide is any one of oligosaccharides represented by the following formulae:
Gal(6S)β1-4GlcNAc(6S)β1-3Gal(6S)β1-4GlcNAc(6S)
Gal(6S)βl-4GlcNAc(6S) and
Gal(6S)βl-4GlcNAc
wherein Gal represents a galactose residue; GlcNAc represents an N-acetylglucosamine residue; 6S means that a hydroxyl group at position 6 is in the form of 6-0-sulfate ester; β1-4 represents a β-1,4-glycosidic bond; and β1-3 represents a β-1,3-glycosidic bond.

7. The therapeutic drug according to any one of claims 1 to 6, wherein the derivative of the keratan sulfate oligosaccharide is an acylated derivative thereof at a hydroxyl group(s).

8. The therapeutic drug according to claim 7, wherein the derivative of the keratan sulfate oligosaccharide is represented by the following formula: 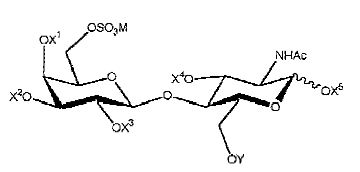 wherein X¹ to X⁵ each independently represent a hydrogen atom or an acyl group; Y represents a hydrogen atom or SO₃M; M represents a hydrogen atom, a mono- to trivalent metal cation, or a mono- to trivalent base; and the bond shown by a wavy line represents a single bond in α-glycosidic configuration or β-glycosidic configuration.

9. The therapeutic drug according to claim 8, wherein all of X¹ to X⁵ are acyl groups having 1 to 10 carbon atoms and M is an alkali metal.

10. The therapeutic drug according to claim 9, wherein the derivative of the keratan sulfate oligosaccharide is represented by the following formula: 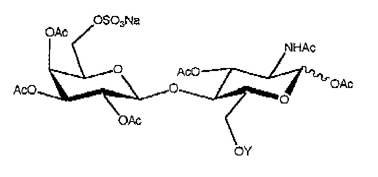 wherein Ac represents an acetyl group; Y represents a hydrogen atom or SO₃Na; and the bond shown by a wavy line represents a single bond in α-glycosidic configuration or β-glycosidic configuration.
